# EUROPEAN PATENT APPLICATION

(11) **EP 4 014 722 A1**
(43) Date of publication of application: **22.06.2022**
(21) Application number: 20214768.2
(22) Date of filing: 16.12.2020
(51) Int. Cl.: A01H 1/06, A01H 5/00

(54) **TARGETED INFLUENCE ON MEIOSIS FOR INCREASING RECOMBINATION FREQUENCY**

(71) Applicant: KWS SAAT SE & Co. KGaA, 37574 Einbeck (DE)
(72) Inventor: Kettles, Nicola, Thriplow, SG8 7RE (GB); Byrne, Edward, Thriplow, SG8 7RE (GB); Mittmann, Sybille, Thriplow, SG8 7RE (GB); Mo, Rui, Thriplow, SG8 7RE (GB); Arrieta, Mikel, Dundee, DD2 5DA (GB); Meade, Claire, Thriplow, SG8 7RE (GB)

(57) **Abstract**

The present invention relates to novel methods for increasing recombination so that the resulting plant or plant material has an increased rate of recombination. In particular, the present invention provides methods for increase of recombination in agricultural plants.

Increased recombination is achieved by a controlled temperature treatment by aligning meiosis progression in a specific treatment window to macroscopically or microscopically detectable characteristics. Furthermore, the methods described herein are also useful to shift meiotic recombination to regions in the genome which usually show a reduced amount of recombination. The resulting plants can for example be used to introduce new genetic variations into breeding pools and breeding populations, as the methods can be performed without a significant loss in fertility usually going along with uncontrolled heat/cold treatments.

## Description

### Technical Field

The present invention relates to novel methods for increasing recombination so that the resulting plant or plant material has an increased rate of recombination. In particular, the present invention provides methods for increase of recombination in agricultural plants. Increased recombination is achieved by a controlled temperature treatment by aligning meiosis progression in a specific treatment window to macroscopically or microscopically detectable characteristics. Furthermore, the methods described herein are also useful to shift meiotic recombination to regions in the genome which usually show a reduced amount of recombination. The resulting plants can for example be used to introduce new genetic variations into breeding pools and breeding populations, as the methods can be performed without a significant loss in fertility usually going along with uncontrolled heat/cold treatments.

### Background of the invention

Plant breeders extensively exploit meiotic recombination to create novel variation and to break deleterious linkages. However, recombination rates are not constant across the chromosome and are generally confined to "hot spots". This leaves large recombinationally cold regions, which are inaccessible to the breeder regarding the introduction of beneficial traits and also genetic mapping.

Meiosis is a central step during the production of gametes and serves the purpose of reducing the chromosome number by half to create reproductive daughter cells having exactly half as many chromosomes. Further, meiosis allows the genomic exchange (crossover) between non-homologous sister chromatids, where recombination plays an outstanding role in allele re-assortment and thus in creating genetic diversity. In flowering plants, male gametophyte develops and reaches maturity in an immature anther.

It is known that meiosis is a complex and cell-specific process proceeding through several stages. Meiosis phase I and phase II are each divided into the same stages, namely prophase, metaphase, anaphase, telophase and cytokinesis, each I and II, respectively. Prophase I is usually the longest phase during meiosis and serves the purpose of allowing homologous recombination (HR) between the paired homologous chromosomes often resulting in a crossover of the chromosomes. Non-sister chromatids may form crossover events, wherein the crossing points are called chiasmata. Prophase I is subdivided into the stages leptotene, zygotene, pachytene, diplotene, and diakinesis.

It is known that certain factors, including shifts in temperature, may alter meiotic progression in relation to the chromosome cycles. This is accompanied by a shift in chiasma distribution with an increase in interstitial and proximal chiasmata. Still, very little generally applicable principles are available. Recombination rates are not constant across chromosomes and are generally confined to specific hot spot regions. Further, in different plants there may be significant differences in the crossover rate between male and female reproductive cells, as this is the case, for example, in Hordeum vulgare (Oshino et al., Molecular Genetics and Genomics, 278:31-42, 2007).

In plants, the change in the recombination profile created by an artificial treatment with various abiotic factors is highly desirable, as an increased rate of recombination, in particular also targeting "cold spots" in the genome, i.e., regions where recombination usually occurs with very low frequencies, is a highly interesting topic. There is the huge obstacle that any intervention in the central meiosis process in plant gametes usually comes along with big losses of fertility making the relevant efforts unpractical for breeding approaches, as fertility of the resulting plant material is key to a desired breeding success. Plants which have new genetic variations due to increase or shift in meiotic recombination and which are infertile cannot be used within breeding. Although they include additional genetic variation these new combinations cannot be transmitted to the next plant generation.

It would thus be desirable to have reliable and efficient protocols available for major crop plants to specifically increase the rate of recombination and to extend recombination to chromosomal cold spot regions to improve and speed up breeding programmes, preferably without the need to use chemicals.

WO 2000/054574 discloses chemical and physical treatments to stimulate recombination. Described is an alteration in chromatin structure due to a heat shock. Still, only a very generic temperature range is provided and there is no guidance at all which further conditions have to be met (time point, exact duration of treatment) to put the observation into practice, as no protocol to reproduce the results is provided. Negative side effects likely to be expected during heat shock treatment of reproductive cells have not been documented and specifically studied. Furthermore, WO 2000/054574 includes a mandatory step of chemical and/or physical treatment of F1 hybrids that result in uncontrolled DNA damage or DNA modifications. Higgins et al. (The Plant Cell, vol. 24: 4096-4109, 2012) studied the overall spatiotemporal asymmetry of the meiotic program in barley and demonstrated that many genes lie within interstitial/proximal regions of low recombination creating big limitations to breeders. It was shown that shifts in temperature may alter meiotic progression in relation to the chromosome cycles in general. This is accompanied by a shift in chiasma distribution with an increase in interstitial and proximal chiasmata. Further, it was stated that heterochromatin may be more accessible for replication factors at higher temperatures. However, the change in the recombination profile observed and exclusively monitored by cytological means came along with big losses of fertility or even sterility, as only a rather crude high temperature heat shock treatment was applied to obtain general data on meiosis progression without aiming at producing material compatible for ongoing breeding projects. Plants which have new genetic variations due to increase or shift in meiotic recombination and which are infertile, however, cannot be used within breeding. Although if they include additional genetic variation, these new combinations cannot be transmitted to the next plant generation.

Hence, there is a great need for an efficient method for increasing the recombination rate or frequency during meiosis and/or for the shift of recombination events during meiosis to regions with low rates of recombination to achieve a rather constant recombination rate across a chromosome of interest during plant breeding. It was thus an object of the present invention to provide methods that should not only allow a shift to increased recombination relying on highly defined mild conditions absent any chemical treatment, wherein the methods simultaneously should necessarily also help to reduce off-target effects of an intervention into the critical process of meiosis. In particular, the methods should not be accompanied by a decreased fertility or even sterility of the relevant plant material and should allow for an increase in recombination of both male and female gametes. Finally, it was also an object of the present invention to provide a time efficient and feasible method for the high throughput treatment of plants.

### Summary of the invention

The present invention was made in view of the prior art described above, and the object of the present invention is to overcome the given deficiencies.

The above object was solved by providing, in a first aspect, a method for increasing meiotic recombination in a plant material, wherein the method comprises the following steps: (a) providing a plant material able to undergo meiosis; (b) identifying a treatment window having a start and an end point based on morphological and/or molecular characteristics specific for the plant material of interest by aligning pre-meiosis and/or meiosis stages to at least one morphological and/or molecular characteristic; (c) optionally synchronizing the plant material; (d) treating the plant material with a diurnal fixed temperature setting for a defined time span based on the start and end point of the treatment window identified in step (b); and (e) obtaining a plant material with increased meiotic recombination in comparison to a control plant material, preferably wherein simultaneously the fertility rate of the plant material having enhanced meiotic recombination is at least 25%, 30%, 40%, 45%, 50%, 55%, 60%, 65%, 70%, 75%, 80%, 85%, 90%, or at least 95% in comparison to a control plant material.

The invention thus has significant relevance to breeding. Plant breeders extensively exploit meiotic recombination to create novel variation and to break deleterious linkages. The invention has the potential to shift recombination into these low-recombining regions and allows the breeder to create new allelic combinations that have been very unlikely to occur before.

To this end, the object of conserving the fertility of the treated plants as much as possible was achieved by providing specific treatment windows and treatment strategies for various target plants, as uncontrolled heat and/or cold treatment would otherwise result in infertile material which cannot be used during breeding.

Further provided is a plant material with enhanced recombination frequencies and uses of the treatment strategies disclosed and claimed to achieve a significant improvement in plant breeding.

The present invention will now further be described based on the attached drawings, the detailed description and the non-limiting examples.

### Brief description of drawings

**Figure 1** (**Fig. 1**) shows a schematic drawing of those settings, where standardized SSD trays were used including matting. The possibility of humidity control and watering is shown by the bottle at the bottom.

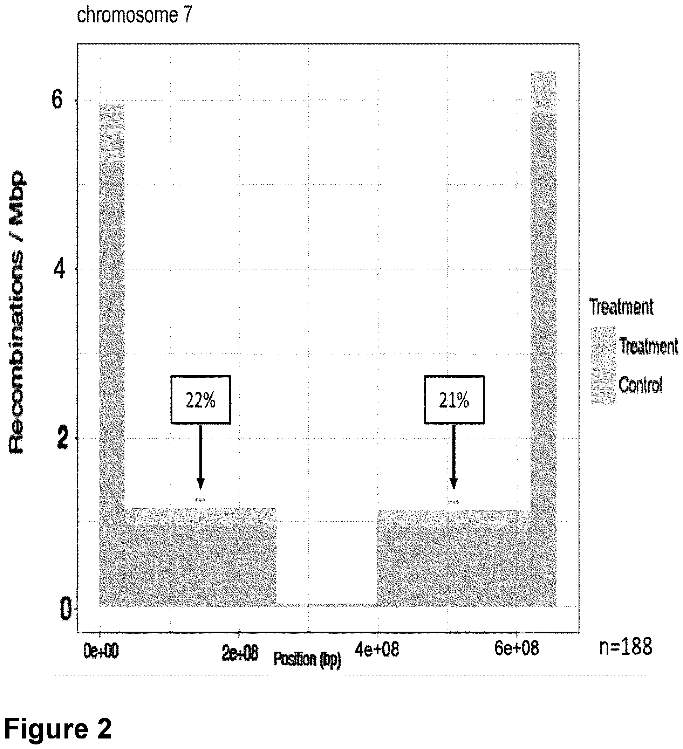

**Figure 2** (**Fig. 2**) shows the exemplary results for a barley genotyping experiment using binomial testing (treatment versus control). The Figure is an output from a binomial test on recombination done with the software "Galaxy" (see also **Example 2** below). Chromosomes were split into zones according to Mascher et al. *supra.* Three zones correlate to low and high recombining regions, whereas Zone 2 is a low-recombining region that was further subdivided. As shown in **Fig**. **2**, Zone 2 showed an increase of recombination if about 21-22% of increase in recombination events from about 200 to 250 in each segment of chromosomes 7H.
**Figure 3** (**Fig. 3**) shows the exemplary results for a barley genotyping experiment using binomial testing (treatment versus control) and further subdividing. The Figure is an output from a binomial test on recombination done with Galaxy (see also **Example 2** below). Here, Zone 2 of **Fig**. **2** was further subdivided into low-recombining (distal) and non-recombining regions (interstitial) for better dissection of the redistribution of crossovers.

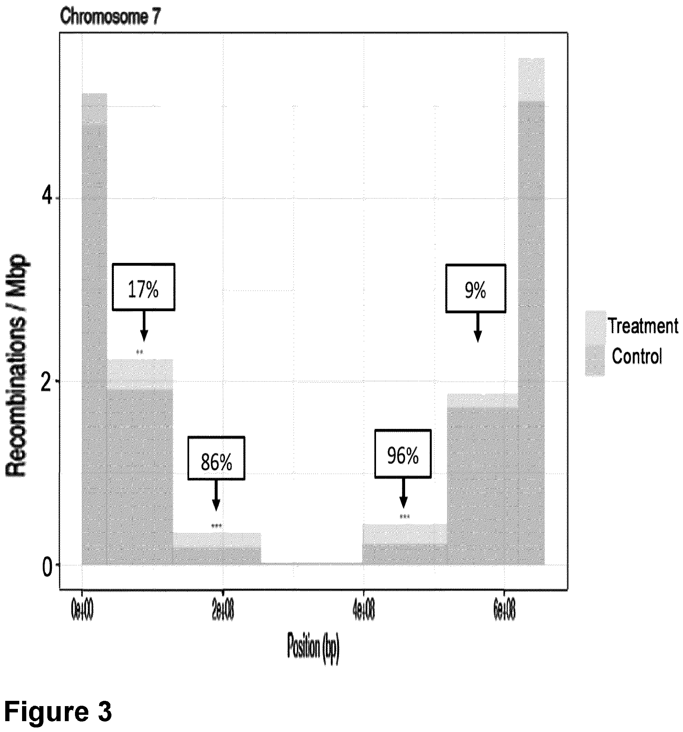

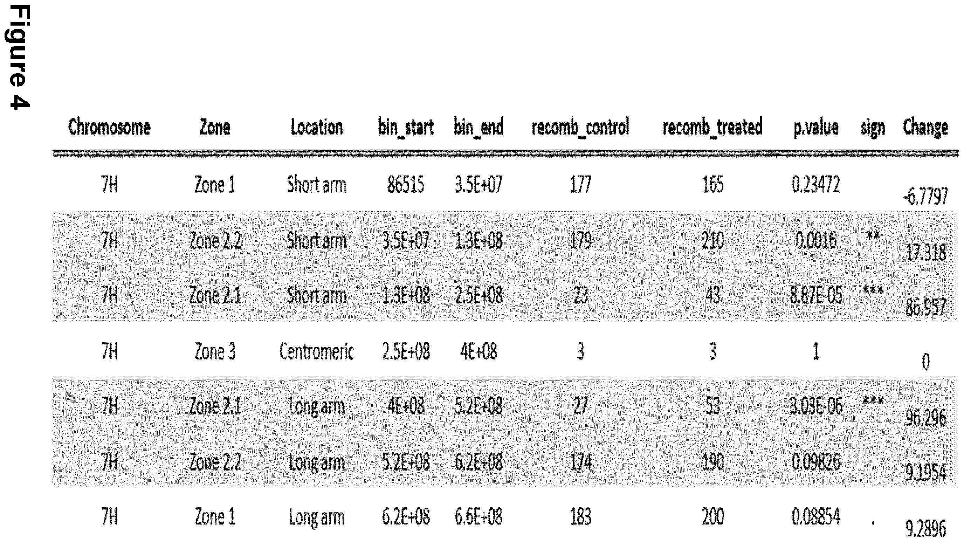

**Figure 4** (**Fig. 4**) (cf. also **Example 2** below and **Fig. 2** and **3** above) shows, in numbers, that the specific temperature treatment as disclosed herein increases crossover events in low-recombining regions from, for example, 27 to 53 crossover events (in Zone 2.1 on the long arm of chromosome 7H of barley, pericentrometric). Zone 2.1 is located besides the centromeric Zone 3, which is the region with the lowest recombination rate. Recombination in Zone 2.1 has been significantly increased by even 86% on chromosome 7H. This data is based in a temperature setting of 28°C to 22°C for the diurnal treatment.
**Figure 5** (**Fig. 5**) shows the results for a specific barely dissection and meiosis staging as further described in **Example 3.**
**Figure 6** (**Fig. 6**) shows the results for determining a treatment window for barley by morphological parameters precisely matched to meiosis progression to generalize the findings (cf. **Example 3**).
**Figure** 7 **(Fig.** 7) shows possible KASP marker for barley (Derived from, 9K iSelect SNP chip) for chromosomes 5 and 7.

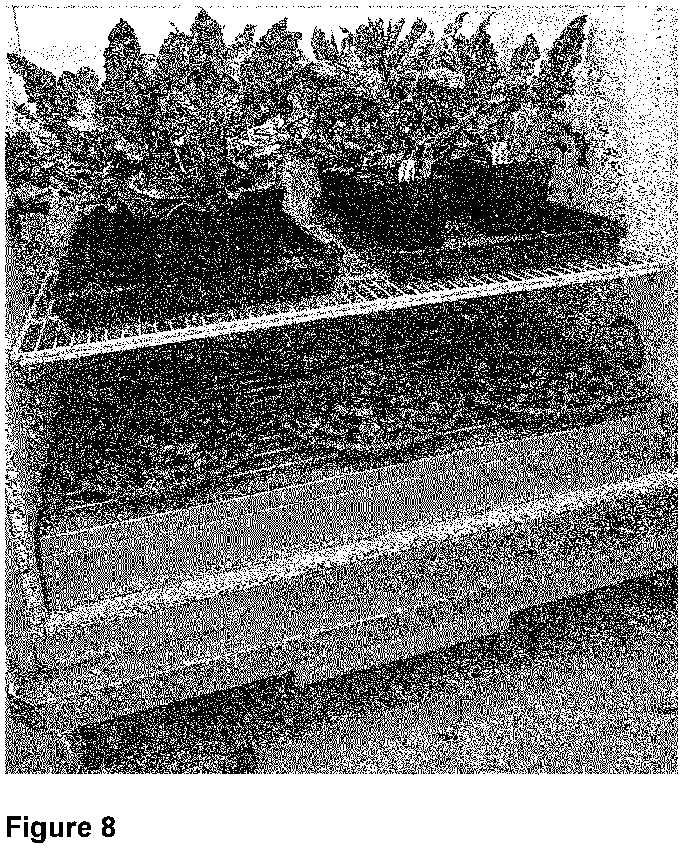

**Figure 8** (**Fig. 8**) shows the humidity control for sugar beet as detailed in **Example 4.**
**Figure 9** (**Fig. 9**) shows the generally applicable sugar beet staging protocol as detailed in **Example 5.**
**Figure 10** (**Fig. 10**) shows microscopic images of meiosis staging for sugar beet its progression. Further, meiosis, tetrad stage and the mature pollen stage after meiosis are precisely matched to the corresponding region in the inflorescence on the right hand side. Further details can be taken from **Example 5.**

### Detailed Description of the Invention

The present invention provides standardisable protocols for a defined heat and/or cold treatment regimen to alter homologous recombination patterns in plants whilst maintaining fertility of the resulting reproductive plant material.

In a first aspect, there is provided a method for increasing meiotic recombination in a plant material, wherein the method may comprise the following steps: (a) providing a plant material able to undergo meiosis; (b) identifying a treatment window having a start and an end point based on morphological and/or molecular characteristics specific for the plant material of interest by aligning pre-meiosis and/or meiosis stages to at least one morphological and/or molecular characteristic; (c) optionally synchronizing the plant material; (d) treating the plant material with a diurnal fixed temperature setting for a defined time span based on the start and end point of the treatment window identified in step (b); (e) obtaining a plant material with increased meiotic recombination in comparison to a control plant material, preferably wherein the obtained plant material is fertile and thus suitable to be directly used in ongoing breeding projects. Preferably, the fertility rate of the plant material having enhanced meiotic recombination is simultaneously at least 25%, 30%, 40%, 45%, 50%, 55%, 60%, 65%, 70%, 75%, 80%, 85%, 90%, or at least 95% in comparison to a control plant material.

The key finding of the present invention is the treatment start and end which has been precisely defined based on a meiosis staging to obtain a treatment window. The time points have been determined to maximise the chance of an effect on recombination while minimizing the effect on fertility, in contrast to other studies which rather arbitrarily trigger meiosis which usually results in sterile plant material. The same can be said about the temperature, which is balanced the same way as further disclosed herein below. A further advantage of the methods is the fact that a successful strategy was defined which uses the start and end point in the conserved meiotic pathway so that these findings can be applied to different plants of interest. From a practical point of view, the meiosis progression relevant for starting and ending a heat or cold treatment was then matched to morphological and/or molecular clues representing characteristics which can be monitored and determined in a standardized manner so that the methods can be easily performed in an ongoing breeding project.

A control plant material having the same origin as the plant material to be subjected to the methods as disclosed herein is usually grown under natural conditions to have an appropriate control.

A plant material according to the present invention is to be understood as any plant cell, tissue, organ (for example, reproductive organs), organelle, seed or whole plant comprising at least one cell able to undergo meiotic cell division during a reproductive cell stage. In angiosperms, meiosis occurs within the cells of the floral organs. Flowers represent the site of meiosis and fertilization in angiosperms. Seeds develop in the context of the ovule following fertilization of the female egg cell by a male sperm cell transferred in pollen, thus dispersal functions are provided both by haploid pollen and diploid seed. Ovules are the site of megasporangium (nucellus) development, which precedes meiosis. Whilst in gymnosperms one to several nucellar cells enter meiosis, in angiosperms a single megaspore mother cell undergoes meiosis to form a tetrad, three members of which degenerate to form a single functional megaspore, which divides mitotically to form the embryo sac, whereas the pollen sac (microsporangium) development occurs from a microsporophyll or in the anther in gymnosperms and angiosperms respectively. In both, sub-epidermal cells are specified as archesporial cells that divide periclinally to form a layer of parietal cells surrounding the sporogenous cells (Harrison et al., Journal of Experimental Botany, vol. 61, p. 2863-2875, 2010; Campbell, The evolution of land plants (embryophyta), 1940, Stanford University Press).

In certain embodiments, the plant material may thus comprise at least one cell or tissue comprising at least one cell able to undergo meiosis selected and optionally isolated from the group consisting of a meristem, including a reproductive meristem, for example, an inflorescence meristem, a spikelet, including at least one floret, including a rachilla, microspores, buds, inflorescence structures, stamens, carpels, anthers, ovaries, megaspore mother cells, microsporangia, gametes, roots, emerged radicles, flowers, flower parts, fruits, pollen, pollen tubes, anther filaments, ovules, embryo sacs, egg cells, ovaries, zygotes, embryos, zygotic embryos, seeds, and cuttings.

Due to the critical character of meiosis in plant reproduction, any intervention into the naturally occurring phenomena, e.g. by applying heat or cold shock treatments or chemicals, is usually associated with significant drawbacks and side-effects.

It was now surprisingly found that these side-effects can be drastically reduced by first providing a defined treatment window and by applying a specific temperature setting to influence meiotic recombination in a favourable way without the risk of losing fertility of the relevant plant material.

A treatment window as used herein refers to a window having a defined start and end point. The identification of the treatment window is dependent on the plant material of interest and may vary. A treatment window can be reliably defined based on morphological, i.e., macroscopically observable, and/or molecular and/or microscopic characteristics as disclosed herein. The identification of the treatment window helps to identify the onset of meiosis based on morphological and/or molecular characteristics specific for the plant material of interest by aligning pre-meiosis and/or meiosis stages to at least one morphological and/or molecular characteristic pre-meiosis stage in a plant material of interest as well as the tetrad stage during meiosis. Therefore, a teaching how to define a start and an end point of the treatment is provided. A tetrad is the foursome structure formed during meiosis made by two homologous chromosomes that have each already replicated into a pair of sister chromatids.

In different embodiments, the exact start and end point of the treatment window may naturally vary. It was found that, for relevant dicotyledonous (dicot) and monocotyledonous (monocot) cultivars, however, the suitable window as such is rather conserved to be suitable for a targeted heat or cold treatment. Even though the duration and onset of meiosis may vary, all plant cells will undergo a tetrad stage which was found to be a reliable end point for the treatment window and thus the end point of the defined and controlled temperature setting. In particular, for barley, sugar beet, maize, canola and wheat the tetrad stage as end point was highly promising. The exact duration of applying the temperature setting will of course vary depending on the cultivar and it was found that the start and end point definition and the matching to observable characteristics is the actually important aspect. Regarding the start point of the treatment, there may be more flexibility. Either, the treatments worked well when starting in early meiosis, but promising data were also achieved when starting in a late mitotic phase, i.e., in pre-meiosis. In all cases, fertile plant cells were obtained. The degree of fertility was found to depend on the start and end point and the fact that a controlled heat or cold treatment in a diurnal scheme was applied. Crude heat and cold shocks and unregularly applied temperature shifts significantly decreased fertility, sometimes even to sterility. It should be noted that the heat shock experiments also led to an increase in meiotic recombination. This phenomenon, however, is useless for breeding purposes in case the resulting plant cells are sterile in the end.

Depending on the plant material of interest, a synchronization may be applied. Synchronization means that the plants or the plant material is treated under standardized conditions so that the skilled person is aware of the fact that synchronizing germination or flowering and more generally a standardized growth and development represents a helpful step in controlled breeding and that, for example, various dicots or monocots, or C3, C4 and CAM plants naturally have different needs regarding synchronization. For instance, some plants may require vernalization, others not and germination or flowering timing is specific for a plant of interest etc. For controlled breeding, and in particular for the methods as disclosed herein, a synchronizing step may thus be highly favourable for certain target plants, but other plants may not need a stringent synchronization, as they may not need vernalization (e.g., *Zea mays*) etc.

In embodiments including vernalization, the defined vernalization cold treatment and duration may thus vary depending on the plant material of interest. The duration may be up from several days to weeks. The vernalization temperature range is typically between around 2°C to 15°C, more typically between around 5°C and 10°C again depending on the target plant of interest. After vernalization, the treated plants or plant materials, they have acquired the ability to flower, or on a molecular stage, in case the plant material is isolated tissues or cells, the ability to start meiotic phases later on.

In certain embodiments, the methods for increasing meiotic recombination may comprise an additional step (f) comprising genotyping the plant material. Genotyping helps to characterize the plant material treated which is of great importance if the material will be further used in ongoing breeding. Nowadays, genomic data and suitable chip sets as well as markers are available and published for all relevant crop plants. Further, statistic tools to verify precisely on which chromosomes an increase in recombination was achieved based on the methods disclosed herein are known to the skilled person and are further exemplified below (for barley, see Mascher et al. (2017, Nature, 544, pp. 427-433)). Genotyping may also be done by any means of molecular biology including PCRs, SNP analyses, transcriptome analysis and the like, preferably by using specific molecular markers.

In certain embodiments, the methods disclosed herein may rely on the cultivation of the plant material in defined modular trays, preferably using defined matting. This can additionally increase the efficiency of enhancing meiotic recombination by having a higher degree of control and synchronisation, where required.

In one embodiment, means for improving the watering of the plants may be provided. The plant materials and plants can thus be cultivated using defined matting. This can additionally help to control impacting relevant processes like meiosis in a precise way. Further, this may help to control the humidity during the ongoing methods which may assist in improving a desired meiotic shift.

As the methods according to the present disclosure do not need any backcrossing steps, they represent a rather streamlined procedure strongly simplifying usually cumbersome and thus time- and cost-intensive settings in breeding.

In one embodiment according to the method of the first aspect, each of the two diurnal fixed temperature settings may comprise a heat treatment at a temperature in the range from about 19°C to about 30°C, wherein the first and the second temperature are different, preferably wherein the temperature is in a range from about 20°C to about 28°C, more preferably from about 20°C to about 26°C. The diurnal fixed temperature setting is thus an artificial temperature setting mimicking a 24 h day/night cycle, but being controlled to allow a targeted manipulation of the meiotic recombination frequency. The setting may be achieved by cultivating the plant material under green house, or under otherwise tightly controllable conditions. According to the present invention, the temperature setting is first determined based on morphological and/or molecular characteristics and then the treatment window is fixed. It may be suitable to keep the temperatures chosen nearly constant as far as reasonably possible during applying the first and the second of the diurnal treatment temperature window.

In another embodiment according to the method of the first aspect, at least one of the two diurnal fixed temperature settings may comprise a cold treatment at a temperature in the range from about 1°C to about 18°C, preferably in the range from about 2°C to 16°C, or from about 5°C to 12°C. As for the heat treatment, the cold treatment may influence the meiotic cycle progression in a targeted way. It is thus important to artificially include a heat or a cold treatment step in a defined manner. Usually, the heat or the cold treatment as relevant trigger may represent the longer of the diurnal time span to achieve optimum results. A time span in the context of a diurnal setting implies the duration of the day, and the duration of the night span chosen, respectively.

In certain embodiments, the methods of the present invention preferably may comprise a diurnal treatment, i.e. a treatment calculated on 24 h in sum, wherein the time span of the diurnal treatment comprises a longer and a shorter time span, wherein the longer time span is about 13 h, about 14 h, about 15 h, about 16 h, about 17 h, about 18 h, about 19 h, about 20 h, or about 21 h.

Based on the knowledge of the skilled person and the disclosure provided herein, it can be understood that the exact time span will vary for different target plants and plant materials. Dependent on whether a heat or a cold treatment is used, the longer time span will usually include the heat and or cold treatment, whereas the short time period, e.g. overnight, will include the regeneration at a second temperature, which may also be termed a resting temperature.

In certain embodiments, an around equivalent 12 h / 12 h scheme may also be chosen. Again, this depends on the target plants and in particular also the delta between the higher and the lower temperature. The more extreme the temperatures chosen for heat or cold treatment, the shorter the time span. In contrast to only scientific settings, where any effect on meiosis is quickly triggered, the methods of the present invention aim at providing fertile material so that severe heat and/or cold shocks will be avoided to obtain fertile plant material.

A temperature setting thus mainly implies the definition of two distinct treatment temperatures as detailed above.

A relevant contribution of the present disclosure is the provision of exact temperature regimens to influence meiotic progressions and to enhance recombination frequency in a targeted way accordingly in a favourable manner. Plants have often been treated under real heat shock conditions above 30°C to study meiosis and the influence on (hetero)chromatin on a scientific scale. These studies, however, aimed at providing basic information on the central meiosis step during reproduction. The resulting plant material, however, is usually less fertile or even sterile and thus unusable for any further breeding or editing steps.

The findings disclosed herein, in particular regarding the treatment window, can be applied to different monocot and dicot plants. The staging for each plant will vary due to the specific growth characteristics, yet the overall teaching regarding the start and end point of the treatment window as disclosed herein as well as the particulars regarding temperature treatment and synchronization can be applied to other plants. Based on the disclosure provided herein, the skilled person can easily adapt the findings herein to relevant dicot and monocot plants of interest.

In one embodiment, the temperature of the longer time span of the diurnal treatment of the methods disclosed herein may thus be higherthan the temperature of the shortertime span of the diurnal treatment.

In another embodiment, the temperature of the longer time span of the diurnal treatment of the methods disclosed herein may be lower than the temperature of the shorter time span of the diurnal treatment for those settings, where meiotic changes are to be triggered by cold treatments or even cold shocks.

In certain embodiments, the start point of the treatment window may be during the pre-meiosis or during or early meiosis stage. Notably, cell cycle progression through mitosis and meiosis is a biological process well known to the skilled person. These processes are essential and are thus rather conserved in different organisms. Even though the duration of the cell cycle and the different stages may strongly vary in different plants, the overall cascade is the same and can easily be determined as it is known in the relevant technical field and as further exemplified herein.

In one embodiment, the end point of the treatment window may be during the tetrad stage of meiosis. A tetrad, as detailed above, is a rather characteristic foursome structure occurring during meiosis. Favourably, this structure can be easily detected and characterised microscopically (see Figure 4).

According to the present invention, not only start and end points for specific heat/cold treatments were defined. Further, the molecular and microscopic events were precisely matched to macroscopically observable characteristics. This drastically improves breeding. Obviously, morphological and/or molecular characteristics can be used for staging. In ongoing breeding settings, it may be particularly favourable to have morphological clues at hand to expedite breeding and to avoid cumbersome and expensive analytics. Nonetheless, a molecular characterization in the developmental phase is important to be able to define the start and end points of the heat or cold treatment individually for a target plant material of interest.

In certain embodiments, morphological and/or molecular characteristics of a plant material may be independently selected from the group consisting of a macroscopic determination of the development of one or more spike(s), the development of the first node, the start of development of the first inflorescence, the length of the leaf collar, anther ear base or ear, a microscopic determination of the onset of meiosis in germ cells of a plant material of interest, or a molecular determination of the onset of meiosis in germ cells of a plant material of interest by transcriptome analysis, or a combination thereof.

(Pre-)Meiotic stages that can be easily detected and thus be used for staging are, for example, the pre-meiosis G2 stage, the leptotene and/or the zygotene as they are rather characteristic. For embodiments relying on microscopy, an anther, e.g. of the middle floret of the most advanced plant/inflorescence, may be squashed and analyzed for the release of the pollen sac. There is a certain probability that G2/pre-meiotic stages are comprised if the anther is less equal or less than 0.5 mm. If it contains cells in leptotene the rest of the florets and plants will still be mostly in pre-meiosis and ready for treatment. The difference between the stages can also be determined based in the size of the nucleus. Leptotene cells usually start to increase in size, and the number of nucleoli is decreasing in leptotene quite generally in plant cells.

According to the present disclosure, a seed may be cultivated, or a plant material may be cultivated under conditions that allow germination and/or progression to meiosis. Growth can proceed, for example, until i) the onset of meiosis in the germ cells, ii) the development of one or more spikes, iii) the first node of the plant becomes detectable, or iv) the first inflorescence starts to develop. For *in vitro* cultures, these conditions may be different.

Then, the plant material is exposed to the specific temperature setting after the individual treatment window has been determined and the plant material is treated each day for at least one hour to a heat or cold treatment until the development of tetrads during meiosis is initiated.

In certain embodiment, the methods may comprise a step of letting the plants mature after the specific treatment according to the present invention.

In certain embodiment, the methods may comprise a step of harvesting plant material.

In one embodiment of the methods disclosed herein, during the whole step (d) or during a part of step (d) the environmental humidity may be increased, preferably the humidity is increased to a value between 60% and 99%, more preferably to a value between 70% and 95%, most preferably to a value between 80% and 92%. The controlled humidity was found to additionally support a targeted manipulation of the meiotic recombination rate.

In embodiments relying on vernalization, the step of synchronization, here as cold synchronization, may comprise an exposition of the plant material to a temperature of between about 5°C to about 18°C, more preferably between about 8°C to about 18°C, for a duration of about 18 to 40 days.

In certain embodiments, the methods of the present invention may comprise a plant material specific staging for identifying a treatment window based on morphological and/or molecular characteristics during step (b). Obviously, this staging will be specific for a target plant or target plant material of interest and actually implies the matching of the molecular findings on meiosis staging disclosed herein to easily traceable morphological clues to speed up breeding.

In certain embodiments, the development of tetrads during meiosis may be indicated either by an emerging flag leaf and/or a spike having a longitudinal axis longer than 2.7 cm and/or an anther having a longitudinal axis no longer than 3 cm.

In other embodiments, the start point of the treatment window may be initiated when the leaf collar of the plant is between about 15 and 25 cm, preferably between about 17 and 23 cm, and more preferably between about 19 and 21 cm.

In another embodiment, the start point of the treatment window may be initiated when the longitudinal axis of the anther is between 0.1 and 1 mm, preferably between 0.2 and 0.9 mm, and more preferably between 0.3 and 0.8 mm.

In another embodiment, the start point of the treatment window may be initiated when the longitudinal axis of the ear base is between 5 and 15 cm, preferably between 7 and 13 cm, more preferably between 9 and 11 cm.

In another embodiment, the start point of the treatment window may be initiated when the ear length is between 0.3 and 1.5 cm, preferably between 0.4 and 1.3 cm, more preferably between 0.5 and 1 cm.

As the skilled person will be aware of, the length specifications will vary dependent on the target plant and are approximations as the measurement is inherently associated with a certain inaccuracy, in particular if the measurement takes place on a living plant material.

In yet another embodiment, the start point of the treatment window may be initiated at the onset of flower development. In case the staging and the identification of the treatment window show that this is a suitable time point where pre- or early meiosis occurs, the parameter of flower development represents a standardized and easy to determine onset.

According to the methods of the present invention, step (d), i.e., the diurnal treatment under a diurnal regime for a specific day/night time span may preferably be performed for a period of about 3 to 24 days, preferably between 5 and 23 days, more preferably between 6 and 22 days and most preferably between 8 and 20 days. This duration of the treatment can vary as the progression of the reproductive and/or vegetative stage differs in various target plants.

In certain embodiments, it may have practical advantages that the shorter second time span of the diurnal treatment, or the resting phase, is performed during the night-time.

In some embodiments, the increased meiotic recombination in comparison to a control plant material according to the methods disclosed herein may be determined by performing a binomial test, preferably by a binomial test including a differentiation between low and high recombining regions in the plant material of interest. Performing such a test can provide highly relevant information as further exemplified in the Examples below. In particular, this can provide information not only on the fact whether meiosis rates are increased, but additionally regarding the fact whether otherwise rather silent cold spots in recombination can also be targeted after performing the methods as disclosed herein.

Depending on the purpose, the plant material as manipulated according to the methods disclosed herein may be used further, actually any increase in meiotic recombination may be desirable. In preferred embodiments, the plant material may have enhanced meiotic recombination in comparison to a control plant material, wherein the meiotic recombination is enhanced by at least 15%, 20%, 25%, 30%, 35%, 40%, 45%, 50%, 55%, 60%, 65%, 70%, 75%, 80%, or at least 85%, wherein simultaneously the fertility rate of the plant material having enhanced meiotic recombination is at least 40%, 45%, 50%, 55%, 60%, 65%, 70%, 75%, 80%, 85%, 90%, or at least 95% in comparison to a control plant material.

It was surprisingly found that the specific diurnal treatment in a treatment window matched to the reproductive meiosis progression does not only yield enhanced recombination frequency, but also a maintenance of fertility. This is in contrast to known protocols applying rather harsh heat or cold shock treatments for just one period, and not fixed temperature setting as disclosed herein following a diurnal rhythm. For plant breeding, this effect is indispensable, as infertile or sterile material is nearly useless for ongoing breeding programmes.

Again depending on the plant material of interest, an increased meiotic recombination rate can be determined by means available to the skilled person. For nearly all cultivars of major interest, linkage mapping is a suitable tool to verify an increase in recombination. It can be applied to all species, for which genomic and optionally marker information is available, even if a full genetic map is not available. Alternatively, cytology and/or microscopy may be used. Crossovers or chiasmata can be visualized by microscopy. Chiasmata represent highly characteristic patterns easily identifiable. Furthermore, specific antibodies specific for meiotic stages may be applied during cytology to visualize crossover events. For example, current meiotic recombination models invoke the use of structure-specific enzymes that symmetrically cleave single Holliday junctions and which are specifically active in and interacting with structures formed during the different stages of meiosis, for example, the endonucleases MLH1 to MLH3 active as mismatch repair factors (Manhart et al., PLOS Biology, doi.org/10.1371/journal.pbio.2001164) so that antibodies against these proteins can be used to specifically identify certain meiotic events.

In most embodiments, the binomial testing and genotyping as disclosed herein for verifying the effectiveness of the methods may be preferable, as this does not only provide information regarding whether meiotic recombination was increased, but additionally provides information on the fact where the recombination has occurred. Therefore, these methods have a higher resolution of the genetic mapping of a successfully enhanced meiotic recombination event.

Likewise, fertility rate can be determined by the methods disclosed herein and by methods well known to the skilled person. For instance, the total number of spikelets or florets in an ear versus the number of spikelets or florets which contain grain at maturity may be expressed as a percentage. As for all methods disclosed herein, plant material treated according to the methods of the present invention is always compared to control plant material.

Especially a plant to be used for agricultural purposes (agricultural plant or crop) is suitable to be treated according to the methods of this invention. The plants may be monocots or dicots. Among the monocots *Zea mays* as well as cereals may be selected. The cereals comprise for example barley, rice, wheat, millet, sorghum, oat, rye, triticale and tricitum. The dicots comprise for example sugar beet, red beet, swiss chard, soybean, rapeseed, sunflower, spinach, asparagus, beans, broccoli, cabbage, carrot, cauliflower, celeriac, celery, cucumber, eggplant, kale, kohlrabi, leek lettuce, melon okra, onion, parsley, peas, pepper, pumpkin, radish, squash, tomato, turnip and water melon.

Preferably, plants which may be subject to the methods and uses of the present invention are plants of the genus selected from the group consisting of Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus. More preferably, the plant is selected from the group consisting of Hordeum vulgare, Hordeum bulbusom, Sorghum bicolor, Saccharum officinarium, Zea spp., including *Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.* Particularly preferred are *Beta vulgaris, Zea mays, Triticum aestivum, Hordeum vulgare, Secale cereale, Helianthus annuus, Solanum tuberosum, Sorghum bicolor, Brassica rapa, Brassica napus, Brassica juncacea, Brassica oleracea, Raphanus sativus, Oryza sativa, Glycine max,* and/or *Gossypium sp.*

Usually and preferably, the methods of the invention are no essentially biological processes. In particular, the methods as disclosed herein are not applied for crossing and selecting plants, but rather to artificially treat a plant material to provide a plant material having a higher recombination frequency. This may be suitable for subsequent breeding steps to improve the introgression of foreign material in a shorter time span by breaking linkage blocks much quicker than relying on traditional methods.

In turn, the methods according to the present invention may be applied *in vivo.* In another embodiment, the methods of the present invention may be applied *in vitro,* for example, on a cell or tissue culture able to undergo meiosis. The staging for tissue cultures may vary. For tissue cultures, in particular microscopic and molecular tools can be applied when determining a treatment window for *in vitro* cultured cells, tissues or organs or plant parts.

In another embodiment, a plant material obtained or obtainable by the methods of the present invention may be used for enhanced genome editing. In view of the fact that any targeted manipulation of a plant genome necessarily relies on the accessibility of the genomic material to be modified to a site-directed nuclease or nickase and optionally a repair template and, for nucleic-acid guided nuclease (e.g. a CRISPR nuclease or a nickase or nuclease-dead variant thereof) also at least one guide RNA, the methods as used herein are highly suitable as a pre-treatment, or a parallel treatment, for achieving better results of site-specific targeted genome editing.

In one aspect, there may be provided a for the targeted modification (insertion, deletion, exchange of nucleotides) of at least one genomic target sequence in a plant cell, wherein the method comprises the following steps: (a) performing a method for increasing meiotic recombination in a plant material according to the main aspect as disclosed herein; (b) introducing into the cell (i) at least one site-specific nuclease, or a sequence encoding the same, and, for a nucleic-acid guided nuclease, at least one guide nucleic acid sequence, or a sequence encoding the same, and optionally at least one repairtemplate, wherein step (b) is performed simultaneously with treating the plant material with a diurnal fixed temperature setting, or wherein step (b) is performed after a plant material with increased meiotic recombination has been obtained; and obtaining at least one modified cell.

The at least one nucleic-acid guided nuclease, or the sequence encoding the same, may be selected from a nuclease from a CRISPR/Cas system, preferably from a CRISPR/Cfp1 system, a CRISPR/MAD7 system, a CRISPR/MAD2 system, a CRISPR/Cas9 system, a CRISPR/CasX system, or a CRISPR/CasY system, wherein the CRISPR/Cpf1 system may be selected from the group consisting of a CRISPR/Cfp1 system from *Lachnospiraceae bacterium* ND2006 (LbCpf1), a CRISPR/Cfp1 system from a *Acidaminococcus* sp. BV3L6 (AsCpf1), or a CRISPR/MAD7 system from *Eubacterium rectale.*

In one aspect the invention concerns plants, plant parts and a plant cell / plant cells treated according to the methods of the present invention. Those plants, plant parts and plant cell(s) are preferably not products of essentially biological processes. This is evident, as the methods of the main aspect disclosed herein do not rely on a step of crossing or selecting. Rather, the methods aim at specifically influencing meiosis rates in a given population and generation of a plant material.

As the main aspect of the present invention provides for the increase of recombination in a plant and/ or for the shift of meiotic recombination to regions in the genome of a plant which usually show a low amount of recombination, there may also be provided the use of these methods and the products obtainable by these methods for enhancing recombination frequency whilst simultaneously reducing off-target effects on fertility, wherein both lead characteristics can be easily monitored and determined based on the disclosure provided herein. This represents a significant improvement for plant breeding.

Allowing the seed to germinate, as disclosed herein for the methods of the present invention, includes all possible measures which trigger the process of germination. It is recommended to moisture the seed with water or to place it in an environment which contains sufficient moisture. Optimally the seed will be placed during the germination process in a suitable substrate. Examples for suitable substrates is soil, filter paper or mineral granules. The substrate should contain sufficient moisture to promote the germination. In most plant species a successful germination is indicated by the emergence of radicles and the cotyledon / cotyledons.

After the germination the seedling should be exposed to sufficient light for promoting the further development of the plant. Constant access to water for the plant is necessary.

To influence the recombination during the meiosis the plant needs to develop a stage where the production of cells able to undergo meiosis, for example, gametic cells within the plant is initiated. The gametic cells may be part of pollen or ovum. The meiosis will occur during the maturation of the gametic cells. The start of this process is herein called onset of meiosis and is dependent on the developmental stage of the plant. During the meiosis homologous chromosomes build chiasmata and recombination of genetic material occurs. This recombination may create new genetic variance. Such new genetic variance may impart beneficial phenotypical properties like for example resistance towards plant pathogens, resistance towards plant herbicides or an increase in the production of desired plant compounds like oil, starch or sugar.

For the purpose of the invention it is important to let the plant grow till the onset of meiosis. The onset of meiosis may be recognized for example on a molecular level wherein cells from the reproductive tissues of the plant are being analysed for their chromatin content. Such an analysis can for example be done using flow cytometry or light microscopy involving chromosome staining. In case of a diploid organism a chromatin content of 1c per cell is interpreted as the result of meiosis.

The expression "development of one or more spikes" preferably the development stage of a plant at which the first spike is developed. To identify the development of one or more spikes the following table according to Witzenberger et al., 1989 and Lancashire et al., 1991 may be used:

**Tab. 1 Phenological growth stages and BBCH-identification keys of cereals**

| Code | Description |
|---|---|
| 0 | Principal growth stage: Germination |
| 1 | Principal growth stage: Leaf development |
| 2 | Principal growth stage: Tillering |
| 3 | Principal growth stage: Stem elongation |
| 4 | Principal growth stage: Booting |
| 4.1 | Early boot stage: Flag leaf sheath extending |
| 4.3 | Mid boot stage: Flag leaf sheath just visibly swollen |
| 4.5 | Late boot stage: Flag leaf sheath swollen |
| 4.7 | Flag leaf sheath opening |
| 4.9 | First awns visible (in awned forms only) |
| 5 | Principal growth stage: Inflorescence emergence, heading |
| 5.1 | Beginning of heading: Tip of inflorescence emerged from sheath, first spikelet just visible |
| 5.5 | Middle of heading: Half of inflorescence emerged |
| 5.9 | End of heading: Inflorescence fully emerged |

The development of the first spike occurs at the development stage according to code 5.1 (s. Tab. 1). The development of one or more spikes occurs between the development stage according to code 5.1 and the development stage according to code 5.5. Preferably, the development of one or more spikes occurs at the development stage according to code 5.1. In this context the terms spike and spikelet are used interchangeably.

In case a plant which belongs to a species that generally has no spikes is to be treated according to the methods herein disclosed the correct development stage of this plant for the application of the heat treatment should be determined by one of the other characteristics as disclosed herein as for example node, inflorescence or the onset of meiosis.

The expression "development of the first node" shall mean that the first note becomes detectable during the development of a plant. In case of cereals the following table of development stages may be used to identify the stag of the development of the first node:

**Tab. 2 Development phases of the stem elongation according Zadoks cereal growth stage key**

| Code | Description |
|---|---|
| GS30 | Pseudo stem erect (Embryo ear at 1cm) - start of stem Elongation: The tip of the developing ear is 1 cm or more from the base of the stem where the lowest leaves attach to the shoot apex. |
| GS31 | 1st node on main stem: The first node can be seen 1 cm or more above the base of the shoot (with clear internode space below it) and the internode above is less than 2 cm. |
| GS32 | 2nd node on main stem - leaf 3 emerges on main stem: The second node can be detected and the internode below it exceeds 2 cm, however the internode space above the node has not yet reached 2 cm |
| GS33 | 3rd node on main stem - leaf 2 (F-1) emerges on main stem |
| GS37 | Flag leaf just visible on main stem |
| GS39 | Flag leaf fully emerged on main stem with ligule showing |

The development of the first spike occurs at the development stage according to code GS31 (s. Tab. 2). During the stage GS31 usually the first node can be seen 1 cm or more above the base of the shoot (with clear internode space below it) and the internode above is less than 2 cm. In case a plant which belongs to a species which is to be treated according to the methods herein disclosed the correct development stage of this plant for the application of the heat or cold treatment should be determined by one of the other characteristics as disclosed herein as for example node, inflorescence or by any molecular and not phenotypically observable characteristics to be able to determine the start point of the treatment window and the suitable end point.

For all embodiments, it is particularly favourable if initially determined molecular characteristics are aligned with morphological clues, i.e., parameters easily verifiable on a macroscopic, phenotypic level.

The expression "first inflorescence starts to develop" shall mean that the development of the first inflorescence in the plant has been initiated. In case of plants belonging to the genus *Beta* (like for example sugar beet, beetroot and chard) the following table of development stages may be used to identify the stage of the development of the first inflorescence:

**Tab. 3 Development phases of beets according to the BBCH-scale**

| Code | Description |
|---|---|
| 0 | Germination |
| 1 | Leaf development (youth stage) |
| 3 | Rosette growth (crop cover) |
| 4 | Development of harvestable vegetative plant parts Beet root |
| 5 | Inflorescence emergence (2nd year of growth) |
| 5.1 | Beginning of elongation of main stem |
| 5.2 | Main stem 20 cm long |
| 5.3 | Side shoot buds visible on main stem |
| 5.4 | Side shoots clearly visible on main stem |
| 5.5 | First individual flower buds on side shoots visible |
| 5.9 | First bracts visible; flower buds still closed |
| 6 | Flowering |

The first inflorescence starts to develop between the stages according to code 5.3 and code 5.9 (s. Tab. 3). Preferably, the first inflorescence develops at stage 5.5. Some plants may need a cold stimulation (vernalization) to develop inflorescence. Among this groups are also plants belonging to the genus *Beta* like sugar beet, beetroot and chard. For the induction of the cold stimulation the plants may be exposed to a temperature of 3 to 5°C after the first season and at the beginning of the second season. The exposure may last for up to six weeks. Preferably, the exposure lasts four, five or six weeks and the preferred temperature are 3, 4 or 5°C.

Regarding plant meiosis and thus the central cell cycle to be modified according to the methods disclosed herein, the skilled person is well aware of the fact that a plethora of reliable methods can be applied. Cytogenetics, i.e., the study of chromosomes, is a well-established discipline and available for all kinds of plant cells. Further, various markers and tools exist for all relevant crop plants to determine the progression of mitosis and/or meiosis.

Part of the invention are also the following embodiments:
[1] A method for increasing the rate of homologues recombination in a plant, comprising the following steps:
   (a) Sow seed under conditions that allow the seed to germinate and to build a plant
   (b) Let the plant grow until one of the following:
      i) onset of meiosis in the germ cells
      ii) development of one or more spikes
      iii) the first node of the plant becomes detectable or
      iv) the first inflorescence starts to develop
   (c) Expose the plants each day for at least one hour to a heat treatment of 23°C - 29°C until the development of tetrads during meiosis is initiated
   (d) Optionally: Let the plants mature
   (e) Optionally: Harvest plant material
   (f) Optionally: Identify at least one plant cell or a seed derived from the plant material having an increased recombination rate in comparison to an untreated plant cell or seed of an untreated plant
[2] The method according to [1] wherein the plants are grown in modular trays.
[3] The method according to [1] or [2] wherein during the whole step (c) or during a part of step (c) the environmental humidity is increased.
[4] The method according to [1] - [3] wherein the plants at step c) are exposed for at least one hour of one day to a temperature of 24 - 28°C.
[5] The method according to [1] - [4] wherein the plants at step c) are exposed for at least one hour of one day to a temperature of 25 - 27°C.
[6] The method according to [1] - [5] wherein between step a) and step c) the plant is exposed to a vernalization step, preferably the vernalization step is between step a) and step b).
[7] The method according to [1] - [6] wherein between step a) and step c) the plant is exposed to an adaptation step during which the plant is exposed to a temperature between 8 and 18°C during 22 to 40 days.
[8] The method according to [1] - [7] wherein step (f) is performed by genotyping.
[9] The method according to [1] - [8] wherein the development of tetrads during meiosis in step c) is indicated either by an emerging flag leaf and / or a spike having a longitudinal axis longer than 2,7cm and / or an anther having a longitudinal axis no longer than 3cm.
[10] The method according to [1] - [9] wherein step c) is initiated when the leaf collar of the plant is between 15 and 25 cm, preferably between 17 and 23 cm and more preferably between 19 and 21cm.
[11] The method according to [1] - [10] wherein step c) is initiated when the longitudinal axis of the anther is between 0,1 and 1mm, preferably between 0,2 and 0,9mm and more preferably between 0,3 and 0,8mm.
[12] The method according to [1] - [11] wherein step c) is initiated when the longitudinal axis of the ear base is between 5 and 15cm, preferably between 7 and 13cm, more preferably between 9 and 11cm.
[13] The method according to [1] - [12] wherein step c) is initiated when the ear length is between 0,3 and 1,5cm, preferably between 0,4 and 1,3cm, more preferably between 0,5 and 1cm.
[14] The method according to [1] - [13] wherein step c) is initiated at the onset of flower development.
[15] The method according to [1] - [14] wherein step c) is performed for a period of 18-24 days, preferably between 19 and 23 days, more preferably between 20 and 22 days.
[16] The method according to [1] - [15] wherein the temperature exposure in step c) is performed for a period of 13 - 19 hours, preferably 14 - 18 hours, more preferably 15 - 17 hours.
[17] The method according to [1] - [16] wherein the plants are additionally to the heat treatment subjected each day to a resting treatment of 18 - 22°C, preferably 19 - 21 °C.
[18]. The method according to [1] - [17] wherein the resting treatment is performed during the time during which the plants are not exposed to the heat treatment.
[19] The method according to [1] - [18] wherein at least a part of the resting treatment is performed during the night-time, preferably wherein all of the resting treatment is performed during the night-time.

The present invention will now be further illustrated by the following non-limiting examples.

### Examples

### Example 1: Barley temperature control protocol

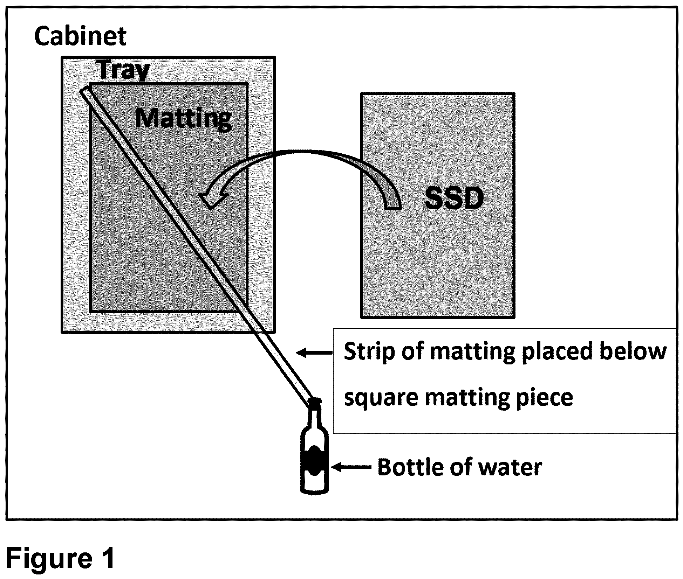

The experiment was conducted to identify the best treatment window for barley. The plant material was cultivated in SSD (single seed descendants) trays using defined matting, which can help to control and standardize the cultivation and thus the determination of the treatment window, the synchronization and the later staging. Water bottles, fertilizers, compost or generic planting soil and tinytag data loggers were used. The strategy of placing the plants on capillary matting is shown in **Figure 1****.**

Next, the sowing followed by germination and vernalization was performed. To this end, SSD trays were filled with regular compost in a slightly compact manner. Seeds were shallowly seeded, wherein the number of seeds depends on the relevant germplasm and whether the starting material is F1 or F2. Water (with fungicide) was used and the plants were allowed to vernalize for about 8 weeks after germination, Then, the trays were moved to a glasshouse.

The subsequent treatment was monitored with data loggers. Once the first node is detectable by non-destructive measures, e.g. feeling with the fingers, in a couple of plants, the trays were moved into pre-programmed growth cabinets (about 2 to 3 weeks after vernalization). Next, the three most mature plants were identified and spikes were extracted and measured. They should all be 0.5 cm or smaller. Then, the plants were moved into a growth cabinet.

Next, various temperature settings were tested in the range from 18°C to 30°C. A diurnal 16 h day and 8 h night phase turned out to be suitable in the present setting, but variations may be possible depending on the plant material. Further, we observed that the day temperature should be higher for the day treatment than for the night treatment to obtain optimum effects on increasing recombination frequencies whilst maintaining fertility. Furthermore, temperature above 29°C or even above 30°C were not suitable in the present setting as this lead to a significant decrease in fertility. Day temperatures of 28°C, 27°C, 26°C, 25°C and 24°C showed promising results to be used as a breeder's tool if combined with an optimum night temperature at least 4°C and preferably at least 6°C lower.

Even though the effect of heat on recombination in barley as an overall phenomenon is known, little to nothing is known about how this could be specifically used to manipulate recombination in an ongoing breeding project in a highly defined way. Depending on the chromosomes investigated, 39% to 74% increase in recombination could be observed by a specific diurnal heat treatment under controlled conditions. These findings are rather significant. As the conditions were controlled and as we could identify clear meiosis markers identifying the start and end point of the treatment (see **Example 3** below), the findings can be extended to other relevant crop plants.

The diurnal temperature setting was applied until the flag leaf was emerging or the spike was above three cm in length for the smallest plants (least advanced) which was again confirmed by feeling the spike inside the stem. The ears that went through treatment were labelled with tape, e.g. spikes >3 cm. The watering was done daily by hand into the trays. Over weekends, water was added to the bottle and the matting strip was put into the bottle after wetting the matting and the strip (cf. **Figure 1**). The continuous watering was relevant for synchronizing the plants and for achieving an optimum treatment.

The plants were fertilized once a week with a standard commercial fertilizer. For maturation, we let the plants grow in the green house. The trays were always left on a bench watering system. For harvest, fertility counts were performed, always per tray to assess variability of sterility between centre and edge of trays. Measurements were taken for the parameters of ear length, grain sites and grain number. All ears were harvested individually.

Finally, analysis was performed. To this end, F2 seeds were sown and leaves were sampled for genotyping. F2 seeds were sent away for genotyping. A binomial test on data using the Galaxy tool was applied for reliable testing.

### Example 2: Barley genotyping and binomial testing

Based on Mascher et al. *supra,* the Galaxy bioinformatic tool was used to pinpoint the zones of the chromosomes where an increased recombination frequency was observed. Chromosome conformation capture for barley is shown in Figure 1 of Mascher et al. *supra.*

The results obtained are shown in **Figures 2** to **4****.** Depending on the treatment window and the temperature regimen chosen a significant increase of crossovers could be observed in naturally low recombining regions on the long arm of, for example, chromosome 7H in Zone 2.1 as particularly evidenced by the findings shown in **Figure 4** showing a significant increase of recombination of even 86%. This data is based on a temperature setting of 28°C to 22°C for the diurnal treatment. Notably, we found even more promising results in case the delta was lower (4°C between heat at regeneration temperature in the diurnal cycle). Still, various settings worked well and recombination was increased by at least 50%, which we regarded to be highly significant, in case the heat treatment was not above 30°C. Most importantly, a delta of 4°C, 5°C, 6°C or 7°C whilst pinpointing the exact start point and the end point in the tetrad step always yielded fairly fertile material. These findings perfectly supported that a targeted treatment of plant material could positively affect the recombination frequency, in particular also to cold spots of recombination, whilst maintaining fertility of the plant material subjected to the protocols established.

First experiments are underway, where a cold treatment is done as the longer time span. Again, exactly the same start and end point as defined for the heat treatment seems to be promising. Not surprisingly, a cold shock treatment should be avoided, but the controlled methods as done for the diurnal meiosis matched heat treatment seem to work fine for barley and sugar beet.

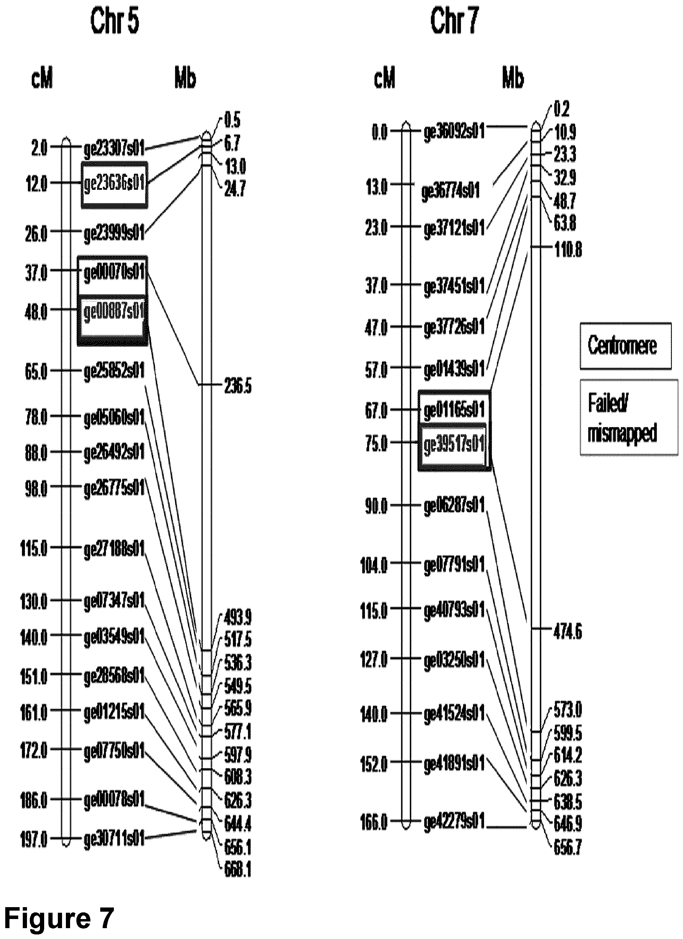

In view of the fact that the first experiments were rather promising, KASP markers were identified for relevant chromosomes to further assist in a streamlined genotyping. The markers can be easily identified for different target germplasm for various target plants based on publicly available information in databases, or the information available for SNP chips. For barley, the 9K iSelect SNP chip was used. The results (chromosomes 5 and 7, shown for barley) are summarized in **Figure 7****.**

### Example 3: Barley staging

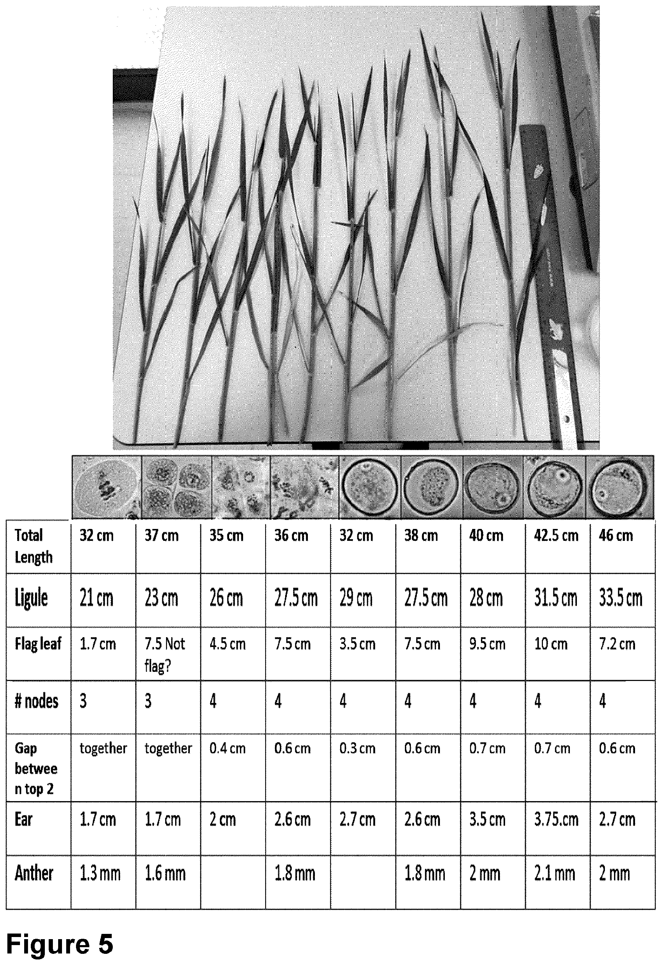

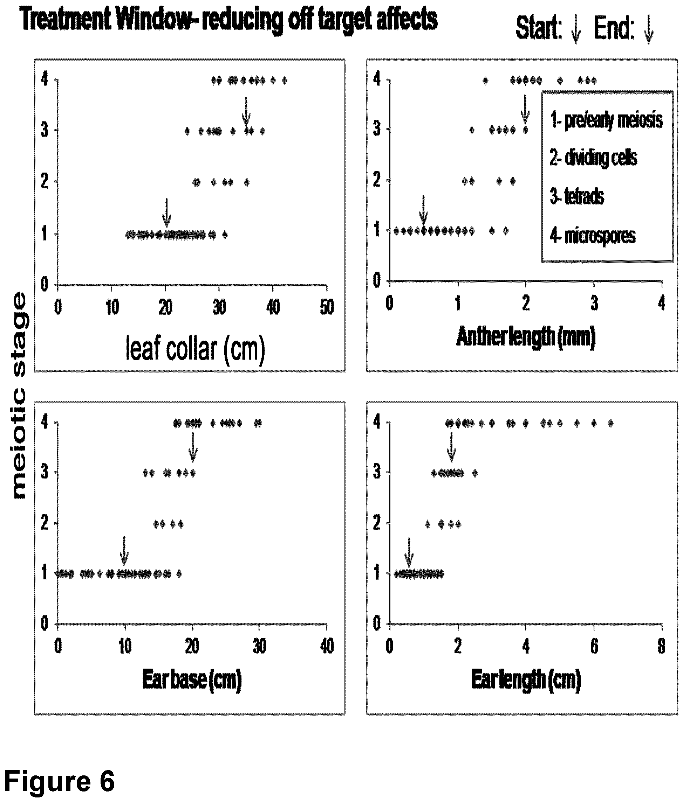

The experiment was conducted to identify the best treatment window to minimize off-target effects such as reduced fertility (or total sterility) to improve and to standardize the methods for enhancing recombination frequency. The plants were grown as previously described for the heat experiment above (**Example 1**). Instead of undergoing treatment they were dissected continuously and assessed for morphological characteristics such as leaf number, height, leaf collar, ear base, spike size, and anther size (**Fig. 5**). These parameters were then specifically linked to the meiotic stage of the plant, where possible (see **Fig. 5** and **6**). The experiment was conducted to identify the best treatment window to minimize off-target effects such as reduced fertility (or even total sterility).

Meiosis staging and plant fixation was done using: a stereomicroscope Microtec HM-4TR; equipped with 10x focusing eyepiece with graticule 10:100 (HM-415FG STG) and HM-3L4N LED stand; a microscope Microtec LM-2TR equipped with wide field eyepiece 10x/FN 20 with graticule 10:100 (LM-E10FG) and calibrating stage micrometer 1 mm in 100 parts (PM-STG) and objective 10x and 40x; dissecting tools with very fine forceps (or insulin needles), and/or a single edge razor blade; glass microscope slides and 18x18 glass coverslip (VWR or similar); aceto-carmine: 0.5g of Carmine (Sigma) were dissolved in 45% acetic acid, it was brought to boil and stirred for 15-20 minutes. Then, the solution was removed from heat, it was cooled down and kept in the fridge overnight. Any precipitates were removed by filtration and the solution was stored in a dark bottle.

Meiosis staging and plant fixation then proceeded as follows:
1. The stems were opened to release the spike (or inflorescence).
2. Even though the morphology of barley and wheat is a bit different, each developing spikelet has three anthers of identical size. Therefore, the protocols can be applied to both cultivars. Under the stereomicroscope, one of the three developing anthers was removed and placed onto a microscope slide.
3. A drop of aceto-carmine was added and squash slightly under a glass coverslip.
4. Under the microscope, the anther was located with a 10X objective and the meiotic stage was identified by using the 40x objectives.

In a series of tests, it was confirmed that the ideal treatment start would be before meiosis, which was found to correspond to an 0.3 cm to 0.5 cm spike in barley, which has been shown to give the best correlation between meiotic stage and morphological parameter besides anther size, which sometimes may need finer dissecting. The end date would be at the end of meiosis II (e.g. matching to the tetrad stage), as this was found to improve fertility. This correlated to an ear size of over 2 to 3 cm. The latter findings seem to be rather important as common heat treatments (without defining when and how long they are applied) to influence meiosis usually reduce fertility or even cause sterility.

### Example 4: Sugar beet temperature treatment

Next, it was an object to find out whether the findings of the monocot barley could be transferred not only to wheat, but also to other relevant dicot plants, like dicot plants belonging to the species *Beta vulgaris.* This species includes important agricultural crops and the following example has been applied to sugar beet.

To have as much as possible standardization, a comparable protocol as detailed above for barely was chosen. Obviously, there are certain differences due to the inherent differences of the target plants. The protocol comprises the following steps:
Sowing the plants into SSD trays (F1 during breeding, or any other plant or plant material); after germination transplantation into 0.1 I pot trays; glass house until the plants reach the four (true) leaf stage; vernalization: 12 weeks (othertime spans may work as well). Notably, it was observed that sugar beet does not necessarily demand a vernalization. The same naturally applies for other target plants like *Zea mays,* the latter originally stemming from more tropical climate zones); adaptation: 11°C for 11 days; potting into 0.75 I pots; optionally: post-adaptation: ~3 weeks at 18°C in the glass house (this may take longer depending on the variety).

In analogy to barely, a treatment window was identified lasting three weeks starting from the first identification of flower development. With the treatment regimens, we first tried lower temperatures starting from 18°C for the night treatment in the diurnal treatment scheme. Temperatures of 20°C or higher seemed to be more promising. Again, the day temperature was chosen higher than the night temperature (4°C to 10°C higher). A delta of around 4°C to 8°C seems to be most promising. Again, it was verified that drastic heat and/or cold shock treatments from the literature (above 30°C, above 34°C or even above 36°C and below 5°C for the cold shock, respectively) were not suitable in case fertile plant material is desired in the end. A cold shock may not be confused with a vernalization temperature. Whilst a cold shock is an abrupt and also (for longer periods) critical condition, vernalization also taking place at very low temperatures is a condition where plants and in particular plant seeds native to temperature regions knowing seasonal temperature shifts may be adapted to.

Humidity was steadily increased during cultivation to avoid drying out prematurely at high temperatures, which will also be beneficial for pollen viability. We added round trays of water and gravel to the bottom of the cabinets to increase humidity.

Next, a bi-weekly fertilization scheme was used. The subsequent post-treatment included the steps: labelling treated flowers with tape e.g. labelling the area of each branch that has finished flowering while undergoing treatment; glass house cultivation until seed matured; watering stop when seed pods became hard; moving to hot glass house, if available; using canes to keep flower stems upright; bagging the stems to make sure they don't break off when they dry.

If plants were not fully dry, but seed pods were mature, the following scheme may be applied: drying at 40°C for 1 to 3 days; testing for readiness (snapping of the stems).

Next, a threshing with bat handle followed and everything was cleaned with a barrel cleaner. If necessary, sifting was used to remove debris. The material obtained was genotyped or sent for genotyping.

### Example 5: Sugar beet staging

For sugar beet staging, inflorescences of sugar beet were dissected and meiocytes were staged with aceto-carmine (similar to barley staging detailed above to obtain standardized protocols). It was identified that inflorescence development is asynchronous and that every stage from pre-meiosis to mature pollen can be found.

It was therefore concluded that treatment should start at the onset of the transition from vegetative to reproductive growth, around the time when an early inflorescence structure can be identified. Sugar beet flowers around 3 to 4 weeks and therefore the treatment window was chosen to be the same as the flowering window based on the very particulars of sugar beet.

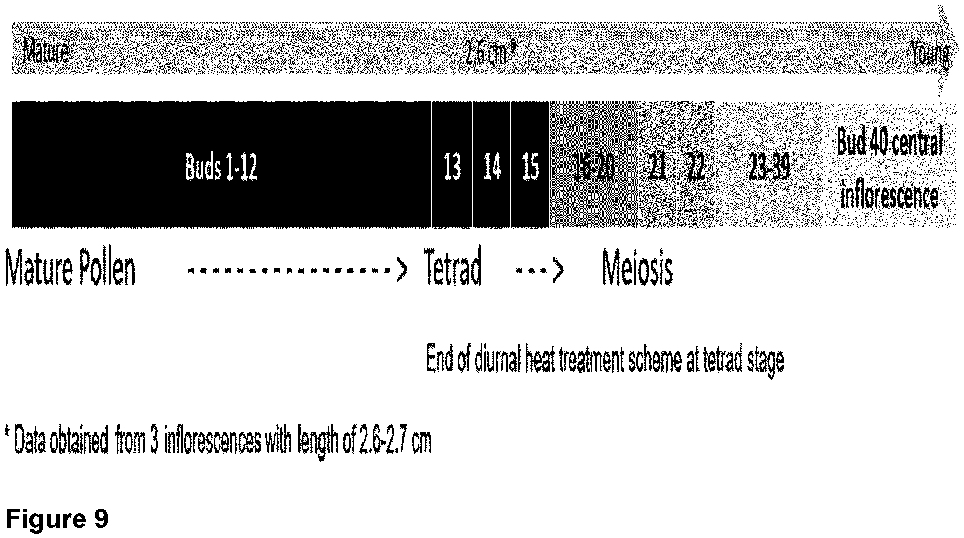

Indeed, it could be affirmed that a comparable treatment scheme as defined for barley also works for sugar beet. After aceto-carmine application, the onset of meiosis, meiosis progression to the tetrad stage and the subsequent mature pollen formation was monitored microscopically. The treatment scheme deduced is shown in **Figure 9****.** Regarding the timing of meiosis is sugar beet, it could be concluded (if comparing different germplasms) that the inflorescence structure was developed before the pre-flower stage. The most mature anther was under meiosis stage when the Inflorescence length was less than 0.5 cm. Therefore, again the microscopic and molecular events as triggered by the specific heat treatment could be aligned to a macroscopic monitoring scheme based on morphological queues to provide an easy tool to be applied during breeding.

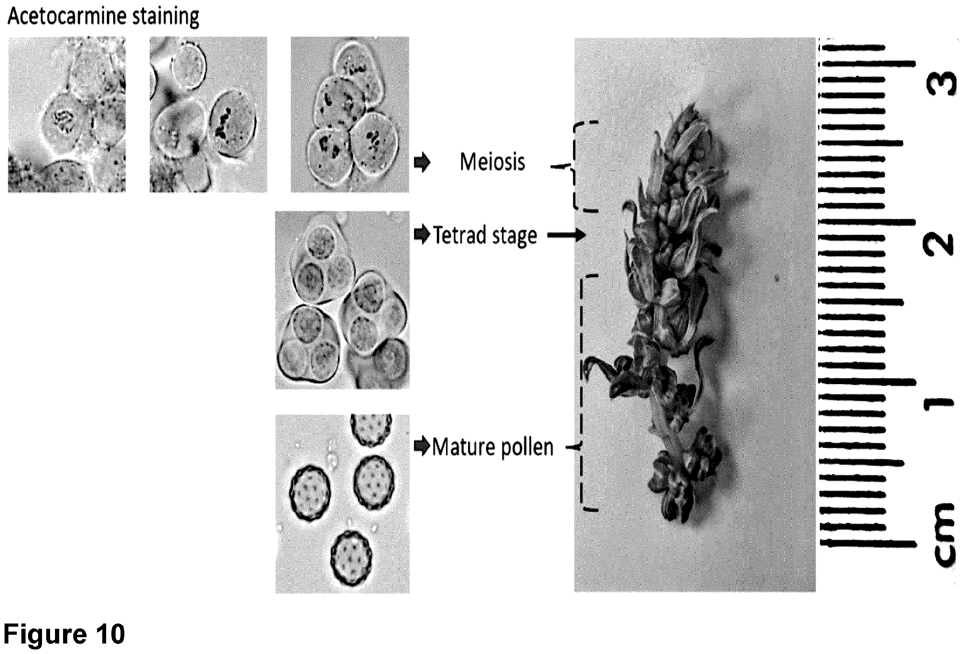

Again it was possible to precisely align the meiosis staging to the macroscopically detectable flowering stage. This is shown in **Figure 10****.** Tetrad stage again served as relevant end point for the treatment.

## Claims

1. A method for increasing meiotic recombination in a plant material, wherein the method comprises the following steps:
(a) providing a plant material able to undergo meiosis;
(b) identifying a treatment window having a start and an end point based on morphological and/or molecular characteristics specific for the plant material of interest by aligning pre-meiosis and/or meiosis stages to at least one morphological and/or molecular characteristic;
(c) optionally synchronizing the plant material;
(d) treating the plant material with a diurnal fixed temperature setting for a defined time span based on the start and end point of the treatment window identified in step (b);
(e) obtaining a plant material with increased meiotic recombination in comparison to a control plant material, preferably wherein simultaneously the fertility rate of the plant material having enhanced meiotic recombination is at least 25%, 30%, 40%, 45%, 50%, 55%, 60%, 65%, 70%, 75%, 80%, 85%, 90%, or at least 95% in comparison to a control plant material.

2. The method according to claim 1, wherein the method comprises an additional step (f) comprising genotyping the plant material.

3. The method according to any of the preceding claims, wherein the method comprises a step (c) of synchronizing, and wherein the synchronisation comprises vernalization and/or a cultivation of the plant material in defined modular trays, preferably using defined matting.

4. The method according to claim 1, wherein each of the two diurnal fixed temperature settings comprises a heat treatment at a temperature in the range from about 19°C to about 30°C, wherein the first and the second temperature are different, preferably wherein the temperature is in a range from about 20°C to about 28°C, more preferably from about 20°C to about 26°C.

5. The method according to any of the preceding claims, wherein the time span of the diurnal treatment comprises a longer and a shorter time span, wherein the longer time span is 12 to 13 h, 13 to 14 h, 14 to 15 h, 15 to 16 h, 16 to 17 h, 17 to 18 h, 18 to 19 h, 19 to 20 h, or 20 to 21 h.

6. The method according to claim 4, wherein the temperature of the longer time span of the diurnal treatment is higher than the temperature of the shorter time span of the diurnal treatment.

7. The method according to any of the preceding claims, wherein the start point of the treatment window is during the pre-meiosis or during or early meiosis stage.

8. The method according to any of the preceding claims, wherein the end point of the treatment window is during the tetrad stage of meiosis.

9. The method according to any of the preceding claims, wherein the morphological and/or molecular characteristics are independently selected from the group consisting of a macroscopic determination of the development of one or more spike(s), the development of the first node, the start of development of the first inflorescence, the length of the leaf collar, anther ear base or ear, a microscopic determination of the onset of meiosis in germ cells of a plant material of interest, or a molecular determination of the onset of meiosis in germ cells of a plant material of interest by transcriptome analysis, or a combination thereof.

10. The method according to any one of the preceding claims wherein during the whole step (d) or during a part of step (d) the environmental humidity is increased, preferably the humidity is increased to a value between 60% and 99%, more preferably to a value between 70% and 95%, most preferably to a value between 80% and 92%.

11. The method according to any of the preceding claims, wherein step (b) comprises a plant material specific staging for identifying a treatment window based on morphological and/or molecular characteristics.

12. The method according to any of the preceding claims, wherein increased meiotic recombination in comparison to a control plant material is determined by performing a binomial test, preferably by a binomial test including a differentiation between low and high recombining regions in the plant material of interest.

13. A plant material obtainable by a method according to any of the preceding claims, the plant material having enhanced meiotic recombination in comparison to a control plant material, wherein the meiotic recombination is enhanced by at least 15%, 20%, 25%, 30%, 35%, 40%, 45%, 50%, 55%, 60%, 65%, 70%, 75%, 80%, or at least 85%, wherein simultaneously the fertility rate of the plant material having enhanced meiotic recombination is at least 40%, 45%, 50%, 55%, 60%, 65%, 70%, 75%, 80%, 85%, 90%, or at least 95% in comparison to a control plant material.

14. The plant material according to claim 13, wherein the plant material is from a plant selected from the group consisting of Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus or wherein more preferably, the plant is selected from the group consisting of Hordeum vulgare, Hordeum bulbusom, Sorghum bicolor, Saccharum officinarium, Zea spp., including *Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.* Particularly preferred are *Beta vulgaris, Zea mays, Triticum aestivum, Hordeum vulgare, Secale cereale, Helianthus annuus, Solanum tuberosum, Sorghum bicolor, Brassica rapa, Brassica napus, Brassica juncacea, Brassica oleracea, Raphanus sativus, Oryza sativa, Glycine max,* and/or *Gossypium sp.*

15. A use of a method according to any of claims 1 to 12 for enhancing recombination frequency whilst simultaneously reducing off-target effects on fertility.
